# EUROPÄISCHE PATENTSCHRIFT

(11) **EP 0 452 693 B1**
(45) Veröffentlichungstag und Bekanntmachung des Hinweises auf die Patenterteilung: **04.10.1995**
(21) Anmeldenummer: 91104410.5
(22) Anmeldetag: 21.03.1991
(51) Int. Cl.: C07C 209/48, C07C 211/09, C08G 69/00, C08G 73/02

(54) **Verfahren zur Herstellung von 2,2-disubstituierten Pentan-1,5-diaminen**
Method of preparing 2,2-disubstituted pentane 1,5-diamines
Procédé pour la production de pentane 1,5-diamines 2,2-disubstitués

(30) Priorität: 30.03.1990 DE 4010252; 07.07.1990 DE 4021726; 28.02.1991 DE 4106340
(43) Veröffentlichungstag der Anmeldung: 23.10.1991
(73) Patentinhaber: BASF Aktiengesellschaft, 67063 Ludwigshafen (DE)
(72) Erfinder: Merger, Franz, Dr., W-6710 Frankenthal (DE); Priester, Claus-Ulrich, Dr., W-6700 Ludwigshafen (DE); Witzel, Tom, Dr., W-6700 Ludwigshafen (DE); Koppenhoefer, Gerhard, Dr., W-6725 Roemerberg (DE); Schuster, Ludwig, Dr., W-6703 Limburgerhof (DE)

(56) Entgegenhaltungen:
- EP-A- 0 010 179
- EP-A- 0 042 119
- DE-A- 1 229 078
- DE-A- 1 817 197
- FR-A- 1 470 245
- FR-A- 2 292 698
- C.R. ACAD. SC. PARIS, Band 268, Serie C, 2. Juni 1969, Seiten 1949-1951; J.-C. MILEO et al.: "Monomères et polymères dérivés du cyano-4-diméthyl-2.2-butyraldéhyde"
- Steric Effects in Organic Chemistry, M. S. Newman Wiley & Sons, 1956, Seiten 467, 468, 598-603, 629-631

## Beschreibung

Die vorliegende Erfindung betrifft ein neues Verfahren zur Herstellung von 2,2-disubstituierten Pentan-1,5-diaminen aus 2,2-disubstituierten 4-Cyano-butanalen.

In C.R. Acad. Sci., Paris, Ser. C 268, 1949-1952 (1969) ist die Herstellung von 2,2-Dimethyl-pentan-1,5-diamin aus 4-Cyano-2,2-dimethylbutanal und aus 2,2-Dimethyl-glutarsäuredinitril beschrieben. Demnach erhält man durch aminierende Hydrierung von 4-Cyano-2,2-dimethyl-butanal in Ethanol in Gegenwart von Raney-Cobalt bei 650 bar 2,2-Dimethyl-pentan-1,5-diamin in 34 % Ausbeute. Das auf sehr aufwendigem Weg durch Umsetzung von 4-Cyano-2,2-dimethylbutanal mit Hydroxylamin und Dehydratisierung hergestellte 2,2-Dimethyl-glutarsäuredinitril wurde in Ethanol bei einem Druck von 200 bar in 70 % Ausbeute in 2,2-Dimethyl-pentan-1,5-diamin überführt. Für eine technische Ausgestaltung des Verfahrens ist die Ausbeute der aminierenden Hydrierung von 4-Cyano-2,2-dimethyl-butanal unzureichend. Darüber hinaus verlangt die Umsetzung einen Druck von 650 bar sowie die Mitverwendung des Lösungsmittels Ethanol. Die Herstellung von 2,2-Dimethyl-pentan-1,5-diamin aus dem entsprechenden Dinitril im technischen Maßstab scheitert vor allem an der Zugänglichkeit des Ausgangsmaterials.

In der DE-A-21 11 765 ist ein Verfahren zur Herstellung von Piperidinen beschrieben, nach dem man N-substituierte 4-Cyanaldimine einer Hydrierung unterzieht unter Bildung von C-substituierten Piperidinen und eines primären Amins, an dessen N-Atom derselbe Substituent gebunden ist "als an das N-Atom des Aldimins". Als Nebenprodukte entstehen, wie aus Seite 3, Zeile 3 bis 6 ersichtlich, geringe Mengen an 1,5-Diaminen. So werden laut Beispiel 1 und 2 aus N-Cyclohexyl-4-cyano-2,2-dimethylbutyraldimin durch Hydrierung an Raney-Nickel bei 120°C und 55 bis 60 bar 6 bis 7 % 2,2-Dimethyl-pentan-1,5-diamin und laut Beispiel 5 analog aus N-Cyclohexyl-4-cyano-2-ethyl-butyraldimin 4 % 2-Ethyl-pentan-1,5-diamin erhalten.

In der DE-A-28 41 585 ist ein Verfahren zur Herstellung von 2,2-Dialkylpentan-1,5-diaminen beschrieben, nach dem in einer ersten Stufe die 4-Cyano-2,2-dialkyl-butanale bei 50 bis 250 bar und 80 bis 160°C in Gegenwart von Metallen der 8. Gruppe des Periodensystems als Katalysator mit Wasserstoff und Ammoniak zum Azomethin aus 4-Cyano-2,2-dialkylbutanal und 4-Cyano-2,2-dialkylbutylamin umgesetzt und in einer zweiten Stufe das entstandene Azomethin aus 4-Cyano-2,2-dialkyl-butanal und 4-Cyano-2,2-dialkylbutylamin bei 50 bis 500 bar und 50 bis 250°C in Gegenwart eines Cobaltkatalysators mit Wasserstoff und Ammoniak zur Reaktion gebracht wird. Wie aus den Beispielen 1 bis 4 ersichtlich, verläuft diese Umsetzung für 4-Cyano-2,2-dimethyl-butanal in unbefriedigender Ausbeute und Raum-Zeit-Ausbeute (Stufe 1: 66,8 % Ausbeute, Stufe 2: 65,2 % Ausbeute, Gesamtausbeute: 43,5 %). Für 4-Cyano-2, 2-diethyl-butanal und 4-Cyano-2-n-butyl-2-ethyl-butanal sinken die Ausbeuten für die erste Stufe auf 46,4 (Beispiel 2) bzw. 57,5 % (Beispiel 3). Die Hydrierung der aus 4-Cyano-2,2-diethyl- und 4-cyano-2-n-butyl-2-ethyl-butanal hergestellten Azomethine ist nicht explizit beschrieben.

In der EP-A-42 119 schließlich ist ein Verfahren zur Herstellung primärer Mono- und Diamine aus Oxoverbindungen, die gegebenenfalls auch weitere zur Reduktion befähigte Gruppen enthalten können, mit Ammoniak und Wasserstoff in Gegenwart bekannter Hydrierkatalysatoren beschrieben, bei dem vor der Reaktion mit Ammoniak und Wasserstoff in Gegenwart von Hydrierkatalysatoren die Oxo-Verbindungen bei Temperaturen von 10 bis 200°C und Drücken von 1 bis 300 bar einer Vorreaktion mit Ammoniak in Gegenwart von anorganischen und organischen Ionenaustauschern in der Ammoniumform als Iminbildungskatalysatoren unterwirft. Die Anwendung des Verfahrens ist ausschließlich in den Beispielen für die aminierende Hydrierung von 3-Cyano-3,5,5-trimethyl-cyclohexanon (Isophoronnitril) und 2,2,6,6-Tetramethyl-4-piperidon (Triacetonamin) beschrieben. Bei der aminierenden Hydrierung von Isophoronnitril werden durch den Einsatz des organischen Ionenaustauschers Lewatit SP® 120 in der Iminierung geringfügige Ausbeuteverbesserungen gegenüber der nicht-katalysierten Fahrweise erzielt (vgl. Vergleichsbeispiel 3 in EP-A-42 119: 90,3 %. Ausbeute, mit Lewatit SP® 120: 93,9 bis 94,7 %.

Es steht also bislang kein Verfahren zur Verfügung, nach dem 4-Cyano-2-ethyl-butanal und 4-Cyano-2,2-dimethyl-butanal unter technisch praktikablen Bedingungen und mit wirtschaftlich befriedigenden Ausbeuten in die entsprechenden Diamine überführt werden können. Pentan-1,5-diamine mit höherem Alkylierungsgrad sind bislang nicht bekannt.

Der vorliegenden Erfindung lag daher die Aufgabe zugrunde, ein Verfahren bereitzustellen, das einen technisch und wirtschaftlich befriedigenden zugang zu 2,2-disubstituierten Pentan-1,5-diaminen aus 4-Cyanobutanalen zu finden und damit auch neue Pentan-1,5-diamine mit höherem Substitutionsgrad in der 2-Stellung herzustellen.

Demgemäß wurde ein neues und verbessertes Verfahren zur Herstellung von 2,2-disubstituierten Pentan-1,5-diaminen der allgemeinen Formel I 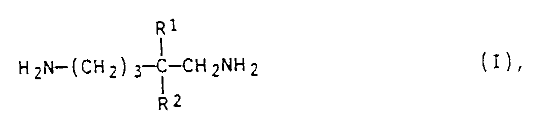
in der die Substituenten R¹ und R² unabhängig voneinander C₁- bis C₁₀-Alkyl, C₂- bis C₁₀-Alkenyl bedeuten oder gemeinsam für eine gegebenenfalls durch eine bis fünf C₁- bis C₄-Alkylgruppen substituierte C₄- bis C₇-Alkylenkette stehen, aus 2,2-disubstituierten 4-Cyanobutanalen der allgemeinen Formel II 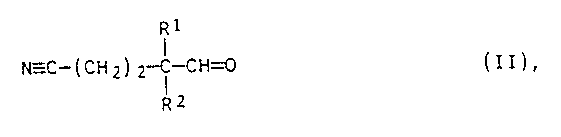
in der die Substituenten R¹ und R² die oben genannten Bedeutungen haben, gefunden, welches dadurch gekennzeichnet ist, daß man in zwei räumlich voneinander getrennten Reaktionsräumen
a) die 4-Cyanobutanale der Formel II in einem ersten Reaktionsraum mit überschüssigem Ammoniak an aciden Heterogenkatalysatoren bei Temperaturen von 20 bis 150°C und Drücken von 15 bis 500 bar umsetzt und
b) die entstandenen Reaktionsprodukte in einem zweiten Reaktionsraum mit überschüssigem Wasserstoff in Gegenwart von überschüssigem Ammoniak an cobalt-, nickel-, ruthenium- und/oder anderen edelmetallhaltigen Katalysatoren, mit basischen Komponenten oder auf basischen oder mit basischen Komponenten auf neutralen Trägern oder basischen Komponenten als Zusatz bei Temperaturen von 60 bis 150°C und Drücken von 50 bis 500 bar hydriert. Ferner wurden die neuen 2,2-disubstituierte Pentan-1,5-diamine 2-Methyl-2-n-propylpentan-1,5-diamine und 2-n-Pentyl-2-n-propylpentan-1,5-diamin gefunden.

Das erfindungsgemäße Verfahren läßt sich wie folgt in zwei räumlich voneinander getrennten Reaktionsräume durchführen:
a) In einer ersten Verfahrensstufe setzt man die 2,2-disubstituierten 4-Cyanobutanale mit überschüssigem Ammoniak um. Dabei hält man einen Druck von 15 bis 500 bar, vorzugsweise von 100 bis 350 bar und eine Temperatur von 20 bis 150°C, vorzugsweise von 30 bis 100°C ein. Die Kondensation wird in Gegenwart von aciden Heterogenkatalysatoren durchgeführt. Als acide Heterogenkatalysatoren eignen sich Metallverbindungen mit Lewissäuren- oder Brönstedtsäure-Charakter wie z.B. Aluminiumoxid, Siliciumdioxid, Titandioxid, Zirkondioxid, ferner Phosphate, wie z.B. Aluminiumphosphate oder Silikate wie z.B. amorphe oder kristalline Aluminosilikate. Bevorzugt verwendet man Aluminiumoxid, Titandioxid, Zirkondioxid und Siliciumdioxid, insbesondere Aluminiumoxid und Titandioxid. Die Acidität der Katalysatoren kann gegebenenfalls durch Dotierung mit Halogeniden erhöht werden. So finden z.B. auch halogendotierte Katalysatoren, wie Chlorid auf Aluminiumoxid oder Chlorid auf Titandioxid, Verwendung.
   Bei der Umsetzung der 2,2-disubstituierten 4-Cyanobutanale an den aciden Heterogenkatalysatoren hält man eine Katalysatorbelastung von 0,01 bis 10, vorzugsweise 0,05 bis 7, besonders bevorzugt von 0,1 bis 5 kg 2,2-disubstituiertem 4-Cyanobutanal pro kg Katalysator und Stunde ein. Pro Mol 2,2-disubstituiertem 4-Cyanobutanal setzt man zweckmäßig, aber nicht zwingend 5 bis 500 Mol NH₃, bevorzugt 10 bis 400, besonders bevorzugt 20 bis 300 Mol ein. Die Umsetzung der 4-Cyanobutanale mit Ammoniak kann auch in der Anwesenheit von inerten Lösungsmitteln, wie Alkanolen oder Tetrahydrofuran erfolgen.
   Die Umsetzung der 2,2-disubstituierten 4-Cyanobutanale läßt sich diskontinuierlich, bevorzugt kontinuierlich durchführen, z.B. in Druckbehältern oder Druckbehälterkaskaden. Nach einer besonders bevorzugten Ausführungsform werden die 2,2-disubstituierten 4-Cyanobutanale und NH₃ durch einen Rohrreaktor geleitet, in dem der Katalysator in Form eines festen Bettes angeordnet ist.
   Die Gesamtverweilzeit in Stufe 1 ergibt sich aus der Katalysatorbelastung und der Menge an eingesetztem Ammoniak. Sie liegt zweckmäßig im Bereich von 0,5 bis 120 Minuten, bevorzugt von 1 bis 40, besonders bevorzugt von 1,5 bis 20 Minuten.
b) Das so erhaltene Produkt wird in einer zweiten Verfahrensstufe mit 3 bis 10.000 Moläquivalenten Wasserstoff, bevorzugt 4,5 bis 30, gegebenenfalls nach Zufuhr von weiterem Ammoniak, einer katalytischen Hydrierung zugeführt.
   Die Hydrierung wird vorzugsweise in flüssigem Ammoniak durchgeführt. Pro Mol in Stufe 1 eingesetztem 2,2-disubstituierten 4-Cyanobutanal verwendet man 5 bis 500 Mol NH₃, bevorzugt 10 bis 400 Mol, besonders bevorzugt 20 bis 300 Mol. Der NH₃-Anteil kann gegebenenfalls durch Zufuhr von NH₃ auf den gewünschten Wert erhöht werden.
   Man hydriert im allgemeinen bei einer Temperatur von 60 bis 150°C, bevorzugt von 70 bis 140°C, besonders bevorzugt von 80 bis 130°C und einem Druck von 50 bis 500 bar, bevorzugt von 100 bis 350 bar, besonders bevorzugt von 150 bis 300 bar.
   Die Katalysatorbelastungen liegen zweckmäßig im Bereich von 0,01 bis 5 kg/[kg·h], bevorzugt bei 0,02 bis 2,5, besonders bevorzugt bei 0,05 bis 2 kg/[kg·h].
   Bei kontinuierlicher Hydrierung ohne Produktrückführung ergibt sich die Gesamtverweilzeit aus der Katalysatorbelastung und der Menge an eingesetztem Ammoniak. Sie liegt im Bereich von 0,5 bis 120 Minuten, bevorzugt von 1 bis 40, besonders bevorzugt bei 1,5 bis 20 Minuten.
   Bei der Hydrierung können prinzipiell alle gängigen Hydrierkatalysatoren eingesetzt werden, die Nickel, Cobalt, Eisen, Kupfer, Ruthenium oder andere Edelmetalle der VIII. Nebengruppe des Periodensystems enthalten. Bevorzugt verwendet man Ruthenium-, Cobalt- oder Nickel-Katalysatoren. Besonders bevorzugt sind Ruthenium- und Cobalt-Katalysatoren. Die katalytisch aktiven Metalle können als Vollkontakte oder auf Trägern eingesetzt werden. Als solche Träger kommen z.B. Aluminiumoxid, Titandioxid, Zirkondioxid, Zinkoxid oder Magnesiumoxid/Aluminiumoxide in Frage, bevorzugt werden Hydrierkatalysatoren mit basischen Komponenten, wie Oxide und Hydroxide von Alkali- und Erdalkalimetallen. Besonders bevorzugt sind daher basische Träger, wie z.B. β-Aluminiumoxid oder Mangesiumoxid/Aluminiumoxide. Besonders bevorzugt ist Magnesiumoxid/Aluminiumoxid mit einem Magnesiumoxidanteil von 5 bis 40 %. Dabei kann der Magnesiumoxid und Aluminiumoxide enthaltende Träger amorph sein oder als Spinell vorliegen.
   Die basische Komponente kann ggf. auch während des Hydrierprozesses zugeführt werden, z.B. als Lösung von Alkali- oder Erdalkalihydroxiden in Wasser.
   Besonders bevorzugt verwendet man in der Hydrierung Cobalt oder Ruthenium mit basischer Komponente. Diese Katalysatoren erhält man technisch in an sich bekannter Weise. So gewinnt man z.B. Ruthenium auf basischem Träger durch Auftragen von wäßrigen Rutheniumsalz-Lösungen wie Rutheniumchlorid und Rutheniumnitrat auf den entsprechenden Träger. Die Ruthenium-Konzentration auf dem Träger beträgt 0, 1 bis 10 %, bevorzugt 0,5 bis 5 %, besonders bevorzugt 1 bis 4 %.
   Nach Trocknung und gegebenenfalls nach Calcinierung bei Temperaturen von 120 bis 500°C, bevorzugt bei 200 bis 400°C, erfolgt die Aktivierung der Ruthenium-Katalysatoren im Wasserstoffstrom bei Temperaturen von 180 bis 250°C, bevorzugt bei 190 bis 230°C und Drücken von 1 bis 500 bar, bevorzugt von 20 bis 300 bar innerhalb von 1 bis 20 Stunden, bevorzugt 2 bis 10 Stunden.
   Die Ruthenium-Katalysatoren können gegebenenfalls noch weitere Metalle enthalten, wie z.B. Palladium oder Eisen. Der Eisengehalt liegt dabei im allgemeinen im Bereich von 0,5 bis 5 %, der Palladium-Gehalt im Bereich von 0,1 bis 5 %.
   Die Umsetzung führt man bevorzugt kontinuierlich, z.B. in druckfesten Rührbehältern oder in einer Rührbehälterkaskade durch. In einer besonders bevorzugten Ausführungsform werden Rohrreaktoren eingesetzt, in denen das Hydriergut in Sumpf- oder Rieselfahrweise über ein fest angeordnetes Katalysatorbett geleitet wird.
   Die Verfahrensstufen a und b können ebenfalls in einem Reaktor durchgeführt werden, in dem Iminierungskatalysatoren und Hydrierkatalysatoren in zwei getrennten Schichten angeordnet sind. In diesem Fall führt man die Iminierung zweckmäßigerweise in Gegenwart von Wasserstoff durch.
   Nach der Hydrierung wird überschüssiges Ammoniak gegebenenfalls unter Druck abgetrennt. Die so erhaltenen 2,2-disubstituierten Pentan-1,5-diaminen (z.B. 2,2-Dimethyl-pentan-1,5-diamin aus 4-Cyano-2,2-dimethyl-butanal, 2-Methyl-2-propyl-pentan-1,5-diamin aus 4-Cyano-2-methyl-2-propyl-butanal oder 2-n-Butyl-2-ethyl-pentan-1,5-diamin aus 4-Cyano-2-butyl-2-ethyl-butanal) lassen sich durch fraktionierende Destillation isolieren. 3-substituierte Piperidine (z.B. 3,3-Dimethylpiperidin aus 4-Cyano-2-methyl-2-propyl-butanal oder 3-Butyl-3-ethylpiperidin aus 4-Cyano-2-butyl-2-ethyl-butanal) entstehen nur in untergeordnetem Maße als Nebenprodukt.

Die Ausgangsstoffe für das Verfahren, die 2,2-disubstituierten 4-Cyanobutanale, sind aus 2,2-disubstituierten Aldehyden und Acrylnitril zugänglich. Das erfindungsgemäße Verfahren ermöglicht erstmals die Umwandlung der 2,2-disubstituierten 4-Cyanobutanale in hohen Ausbeuten und Raum-Zeit-Ausbeuten zu den 2,2-disubstituierten Pentan-1,5-diaminen.

Die Substituenten R¹ und R² in den Verbindungen I und II haben folgende Bedeutungen:
R¹, R² unabhängig voneinander
- C₁- bis C₁₀-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₂- bis C₁₀-Alkenyl, bevorzugt C₂- bis C₈-Alkenyl, besonders bevorzugt C₂- bis C₄-Alkenyl wie Allyl und Butenyl-1
- gemeinsam für eine gegebenenfalls durch eine bis fünf C₁- bis C₄-Alkylgruppen substituierte C₄- bis C₇-Alkylenkette wie -(CH₂)₄-, -(CH₂)₅-, -(CH₂)₆-, -(CH₂)₇- und -CH(CH₃)-(CH₂)₃-.

Bevorzugte 2,2-disubstituierte 4-Cyanobutanale der allgemeinen Formel II sind z.B. 2,2-Dimethyl-4-cyanobutanal, 2-Methyl-2-propenyl-4-cyanobutanal, 2-Ethyl-2-butenyl-4-cyanobutanal, 2-Methyl-2-propyl-4-cyanobutanal und 2-Ethyl-2-butyl-4-cyanobutanal, 2-Methyl-2-nonyl-4-cyano-butanal, 1-Cyanoethyl-cyclohexan-carboxaldehyd, 1-Cyanoethyl-cyclopentan-carboxaldehyd. Diese Ausgangsmaterialien sind aus Isobutyraldehyd, 2-Methyl-pentanal, 2-Methyl-pentenal, 2-Ethyl-hexanal, 2-Ethyl-hexenal, 2-Methyl-undecanal, Cyclohexancarbaldehyd und Cyclopentancarbaldehyd problemlos zugänglich. Bevorzugte 2,2-disubstituierte Pentan-1,5-diamine der allgemeinen Formel I sind 2,2-Dimethyl-pentan-1,5-diamin, 2-Methyl-2-propyl-pentan-1,5-diamin, 2-Methyl-2-propenyl-pentan-1,5-diamin 2-Ethyl-2-n-butyl-pentan-1,5-diamin. 2-Ethyl-2-n-butenyl-pentan-1,5-diamin, 2-Methyl-2-nonyl-pentan-1,5-diamin, 1-(3-Aminopropyl)-1-aminomethyl-cyclohexan und 1-(3-Aminopropyl)-1-aminomethyl-cyclopentan.

Bevorzugte neue Verbindungen I sind:
2-Methyl-2-n-propyl-pentan-1,5-diamin
2-n-Pentyl-2-propyl-pentan-1,5-diamin.

Die beanspruchten Diamine zeichnen sich gegenüber den bekannten Alkylpentandiaminen, wie 2-Methyl- und 2,2-Dimethylpentandiamin, auf Grund des höheren Substitutionsgrades durch geringere Flüchtigkeit und stärkere Asymmetrie (unterschiedliche Reaktivität der Aminfunktionen) aus. Daraus resultieren u.a. leichte Verarbeitbarkeit der Diamine, beispielsweise als Komponenten (Härter) für Epoxide und Komponenten für Polyamide und geringere Geruchsbelästigungen durch unumgesetzte Diamine sowie der daraus herstellbaren Diisocyanate.

### Beispiele

### Beispiel 1

Ein vertikaler Rohrreaktor (Durchmesser: 16 mm, Füllhöhe: 50 cm, ölbeheizter Doppelmantel) wurde mit 81,9 g (93 ml) eines Katalysators mit 3 % Ruthenium auf einem Magnesiumoxid/Aluminiumoxid-Träger (10 % MgO, 90 % Al₂O₃) in Form von 1 bis 1,5 mm Split gefüllt (Katalysatorherstellung durch Porentränkung eines Magnesiumoxid/Aluminiumoxid-Trägers mit wäßriger Ruthenliumnitrat-Lösung und Trocknen bei 120°C). Der Katalysator wurde zur Reduktion bei 100 bar unter gleichzeitigem Durchleiten von 100 Nl/h Wasserstoff nach schrittweiser Erhöhung der Temperatur von 100 auf 220°C innerhalb von 6 h 7 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 63,5 g (100 ml) TiO₂ (Anatas) in Form von 1,5-mm-Strängen gefüllt war, wurde bei einem Druck von 250 bar und einer Temperatur von 60°C stündlich 34,0 g 2,2-Dimethyl-4-cyano-butanal (Reinheit: 93,4 %, 31,8 g, 0,254 mol) und 1450 ml (870 g, 51,1 mol) flüssiges Ammoniak gepumpt. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Der Austrag aus 32,4 Stunden wird durch fraktionierende Destillation über eine 30 cm Füllkörperkolonne (3 mm Glasringe) aufgetrennt. Man erhält 344,8 g 3,3-Dimethyl-piperidin (Kp₂₁₀ = 50-52°C) und 753,6 g 2,2-Dimethyl-pentan-1,5-diamin (Kp₈ = 72°C). Die Diamin-Ausbeute beträgt 70,5 % d. Th.

### Beispiel 2

Beispiel 1 wird mit 2-Methyl-2-propyl-4-cyano-butanal als Edukt wiederholt. Durch den Iminierungsreaktor wurden bei einem Druck von 250 bar und einer Temperatur von 60°C stündlich 33,5 g 2-Methyl-2-propyl-4-cyanobutanal (Reinheit: 88,9 %, 29,8 g, 0,195 mol) und 1400 ml (840 g, 49,4 mol) flüssiges Ammoniak gepumpt. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Der Austrag aus 37,5 Stunden wird durch fraktionierende Destillation über eine 30 cm Füllkörperkolonne (3 mm Glasringe) aufgetrennt. Man erhält 277,0 g 3-Methyl-3-propyl-piperidin (Kp₂ = 46°C) und 842,1 g 2-Methyl-2-propyl-pentan-1,5-diamin (Kp₂ = 78-81°C). Die Diamin-Ausbeute beträgt 72,9 % d. Th.

### Beispiel 3

Beispiel 2 wird wiederholt, wobei durch den Iminierungsreaktor stündlich bei einem Druck von 250 bar und einer Temperatur von 60°C 55,9 g 2-Methyl-2-propyl-4-cyano-butanal (Reinheit: 86,6 %, 48,4 g, 0,316 mol) und 1365 ml (819 g, 48,2 mol) flüssiges Ammoniak gepumpt werden. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Der Austrag aus 16,8 Stunden wird durch fraktionierende Destillation über eine 30 cm Füllkörperkolonne (3 mm Glasringe) aufgetrennt. Man erhält 174,4 g 3-Methyl-3-propyl-piperidin und 501,6 g 2-Methyl-2-propyl-pentan-1,5-diamin. Die Diamin-Ausbeute beträgt 59,6 % d. Th.

### Beispiel 4

Beispiel 3 wird wiederholt, wobei durch den Iminierungsreaktor stündlich bei einem Druck von 250 bar und einer Temperatur von 60°C 78,5 g 2-Methyl-2-propyl-4-cyano-butanal (Reinheit: 86,6 %, 68,0 g, 0,444 mol) und 1340 ml (804 g, 47,3 mol) flüssiges Ammoniak gepumpt werden. Der Austrag wird anschließend bei einem Druck von 250 bar und einer Temperatur von 120°C unter gleichzeitigem Durchleiten von 150 Nl/h (6,7 mol) Wasserstoff von unten nach oben durch den Hydrierreaktor geleitet. Nach Entspannen auf Normaldruck wird NH₃ abdestilliert. Der Austrag aus 4,2 Stunden wird durch fraktionierende Destillation über eine 30 cm Füllkörperkolonne (3 mm Glasringe) aufgetrennt. Man erhält 96,0 g 3-Methyl-3-propyl-piperidin und 162,8 g 2-Methyl-2-propyl-pentan-1,5-diamin. Die Diamin-Ausbeute beträgt 55,1 % d. Th.

### Beispiel 5

Beispiel 1 wird mit 2-Butyl-2-ethyl-4-cyano-butanal als Edukt wiederholt. Dazu wurden durch den Iminierungsreaktor bei einem Druck von 250 bar und einer Temperatur von 60°C stündlich 33,6 g 2-Butyl-2-ethyl-4-cyano-butanal (Reinheit: 89,0 %, 29,9 g, 0,165 mol) und 1344 ml (806 g, 47,4 mol) flüssiges Ammoniak gepumpt. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Der Austrag aus 16,7 Stunden wird durch fraktionierende Destillation über eine 30 cm Füllkörperkolonne (3 mm Glasringe) aufgetrennt. Man erhält 166,8 g 3-Butyl-3-ethyl-piperidin (Kp₂ = 73 bis 75°C) und 267,5 g 2-Butyl-2-ethyl-pentan-1,5-diamin (Kp₂ = 105°C). Die Diamin-Ausbeute beträgt 52, 1 % d. Th.

### Beispiel 6

Ein vertikaler Rohrreaktor (Durchmesser: 16 mm, Füllhöhe: 50 cm, ölbeheizter Doppelmantel) wurde mit 90,1 g (87 ml) eines Katalysators mit 3 % Ruthenium auf β-Aluminiumoxid in Form von 1,2 mm-Strängen gefüllt (Katalysatorherstellung durch Porentränkung von β-Aluminiumoxid mit wäßriger Rutheniumnitrat-Lösung und Trocknen bei 120°C). Der Katalysator wurde zur Reduktion bei 100 bar unter gleichzeitigem Durchleiten von 150 Nl/h Wasserstoff nach schrittweiser Erhöhung der Temperatur von 100 auf 220°C innerhalb von 7 h 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 43,3 g (96 ml) eines mit Aerosil 200 verstrangten Y-Zeolithen gefüllt war (HY-Zeolith:Aerosil 200 = 9:1, SiO₂:Al₂O₃ = 6:1), wurden bei einem Druck von 250 bar und einer Temperatur von 50°C stündlich 41,6 g 2-Butyl-2-ethyl-4-cyano-butanal (Reinheit: 90 %, 37,4 g, 0,207 mol) und 1073 ml (646 g, 38,0 mol) flüssiges Ammoniak gepumpt. Der Austrag wird anschließend bei einem Druck von 250 bar und einer Temperatur von 120°C unter gleichzeitigem Durchleiten von 80 Nl/h (3,6 mol) Wasserstoff von unten nach oben durch den Hydrierreaktor geleitet. Nach Entspannen auf Normaldruck wird NH₃ abdestilliert. Der Austrag aus 34,1 Stunden wird durch fraktionierende Destillation über eine 30 cm Füllkörperkolonne (3 mm Glasringe) aufgetrennt. Man erhält 358,3 g 3-Butyl-3-ethyl-piperidin und 747,7 g 2-Butyl-2-ethyl-pentan-1,5-diamin. Die Diamin-Ausbeute beträgt 56,9 % d. Th.

### Beispiel 7

Ein vertikaler Rohrreaktor (Durchmesser: 16 mm, Füllhöhe: 50 cm, ölbeheizter Doppelmantel) wurde mit 90,1 g (87 ml) eines Katalysators mit 3 % Ruthenium auf β-Aluminiumoxid in Form von 1,2-mm-Strängen gefüllt (Katalysatorherstellung durch Porentränkung von β-Aluminiumoxid mit wäßriger Rutheniumnitrat-Lösung und Trocknen bei 120°C). Der Katalysator wurde zur Reduktion bei 100 bar unter gleichzeitigem Durchleiten von 150 Nl/h Wasserstoff nach schrittweiser Erhöhung der Temperatur von 100 auf 220°C innerhalb von 7 h 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 43,3 g (96 ml) eines mit Aerosil 200 verstrangten Y-Zeolithen gefüllt war (HY-Zeolith:Aerosil 200 = 9:1, SiO₂:Al₂O₃ = 6:1), wurden bei einem Druck von 250 bar und einer Temperatur von 50°C stündlich 45,1 g 2-Butenyl-2-ethyl-4-cyano-butanal (Reinheit: 64,0 %, 28,9 g, 0,161 mol) und 950 ml (570 g, 33,5 mol) flüssiges Ammoniak gepumpt. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Aus dem Austrag von 23,9 Stunden werden durch fraktionierende Destillation über eine 30 cm Füllkörperkolonne (3 mm Glasringe) 205,7 g 3-Butyl-3-ethyl-piperidin und 438,4 g eines Gemisches aus 2-Butyl- und 2-Butenyl-2-ethyl-pentan-1,5-diamin (8:1) isoliert entsprechend einer Diaminausbeute von 61,9 % d. Th.

### Beispiel 8

Ein vertikaler Rohrreaktor (Durchmesser: 16 mm, Füllhöhe: 100 cm, ölbeheizter Doppelmantel) wurde mit 354 g (200 ml) eines basischen Cobalt-Vollkontaktes (CoO mit 5 % Mn₂O₃, 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 20 cm, ölbeheizter Doppelmantel), der mit 25,4 g (40 ml) TiO₂ (Anatas) in Form von 1, 5-mm-Strängen gefüllt war, werden bei einem Druck von 250 bar und einer Temperatur von 80°C stündlich von unten nach oben 80,0 g 2-Butyl-2-ethyl-4-cyano-butanal (Reinheit: 99,3 %, 0,44 mol) und 200 g (330 ml, 11,76 mol) flüssiges Ammoniak gepumpt. Anschließend werden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wird der Ammoniak abdestilliert. Der Hydrieraustrag enthält laut gaschromatographischer Analyse 89,2 % 2-Butyl-2-ethyl-pentan-1,5-diamin und 4,3 % 3-Butyl-3-ethyl-piperidin. Der Austrag aus 80,5 Stunden wird durch fraktionierende Destillation über eine 30 cm Füllkörperkolonne (3 mm Glasringe) aufgetrennt. Man erhält 5738 g 2-Butyl-2-ethyl-pentan-1,5-diamin, entsprechend einer Ausbeute von 87,3 % d.Th.

### Beispiel 9

Ein vertikaler Rohrreaktor (Durchmesser: 16 mm, Füllhöhe: 50 cm, ölbeheizter Doppelmantel) wurde mit 176,7 g (100 ml) eines basischen Cobalt-Vollkontaktes (CoO mit 5 % Mn₂O₃, 1,4 % Na₂O) in Form von 1 bis 1,5 mm Split gefüllt. Zur Reduktion des Kataysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 70,0 g (100 ml) γ-A₂O₃ in Form von 1, 5-mm-Strängen gefüllt war, werden bei einem Druck von 250 bar und einer Temperatur von 80°C stündlich von unten nach oben 20,0 g 2-Butyl-2-ethyl-4-cyano-butanal (Reinheit: 99,3 %, 0,11 mol) und 140 g (230 ml, 8,23 mol) flüssiges Ammoniak gepumpt. Anschließend werden stündlich 60 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wird der Ammoniak abdestilliert. Der Hydrieraustrag enthält laut gaschromatographischer Analyse 95,4 % 2-Butyl-2-ethyl-pentan-1,5-diamin und 2,3 % 3-Butyl-3-ethyl-piperidin. Der Austrag aus 48,0 Stunden wird durch fraktionierende Destillation über eine 30 cm Füllkörperkolonne (3 ml Glasringe) aufgetrennt. Man erhält 920 g 2-Butyl-2-ethyl-pentan-1,5-diamin, entsprechend einer Ausbeute von 93,9 % d.Th.

## Patentansprüche

1. Verfahren zur Herstellung von 2,2-disubstituierten Pentan-1,5-diaminen der allgemeinen Formel I 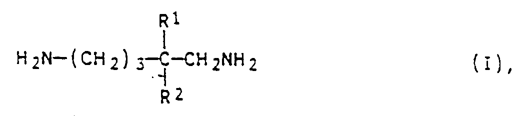 in der die Substituenten R¹ und R² unabhängig voneinander C₁- bis C₁₀-Alkyl, C₂- bis C₁₀-Alkenyl bedeuten oder gemeinsam für eine gegebenenfalls durch eine bis fünf C₁- bis C₄-Alkylgruppen substituierte C₄-bis C₇-Alkylenkette stehen, aus 2,2-disubstituierten 4-Cyanobutanalen der allgemeinen Formel II 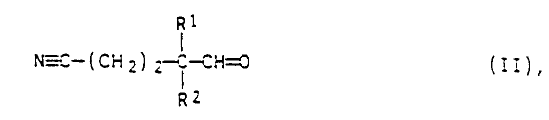 in der die Substituenten R¹ und R² die oben genannten Bedeutungen haben, dadurch gekennzeichnet, daß man in zwei räumlich voneinander getrennten Reaktionsräumen
a) die 4-Cyanobutanale der Formel II in einem ersten Reaktionsraum mit überschüssigem Ammoniak an aciden Heterogenkatalysatoren bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an cobalt-, nickel-, ruthenium- und/oder anderen edelmetallhaltigen Katalysatoren mit basischen Komponenten oder auf basischen Trägern oder mit basischen Komponenten auf neutralen Trägern oder basischen Komponenten als Zusatz während des Hydrierprozesses bei Temperaturen von 60 bis 150°C und Drücken von 50 bis 500 bar hydriert.

2. 2-Methyl-2-n-propyl-pentan-1,5-diamin.

3. 2-n-Pentyl-2-n-propyl-pentan-1,5-diamin.

## Claims

1. A process for the preparation of a 2,2-disubstituted pentane-1,5-diamine of the formula I 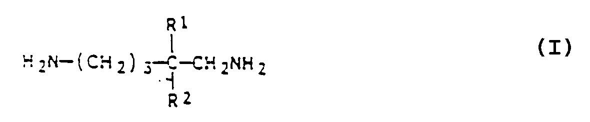 where R¹ and R², independently of one another, are C₁- to C₁₀-alkyl or C₂- to C₁₀-alkenyl or together are a C₄- to C₇-alkylene chain which is unsubstituted or monosubstituted to pentasubstituted by C₁- to C₄-alkyl, from a 2,2-disubstituted 4-cyanobutanal of the formula II 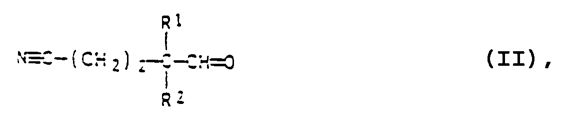 where R¹ and R² are as defined above, which comprises, in two spatially separate reaction spaces,
a) reacting the 4-cyanobutanal of the formula II, in a first reaction space, with excess ammonia on an acidic heterogeneous catalyst at from 20 to 150°C and at from 15 to 500 bar, and
b) hydrogenating the resultant reaction product, in a second reaction space, using hydrogen in the presence of excess ammonia on a catalyst containing cobalt, nickel, ruthenium and/or another noble metal, with a basic component or on a basic carrier or with a basic component on a neutral carrier or a basic component as additive during the hydrogenation process, at from 60 to 150°C and at from 50 to 500 bar.

2. 2-Methyl-2-n-propylpentane-1,5-diamine.

3. 2-n-Pentyl-2-n-propylpentane-1,5-diamine.

## Revendications

1. Procédé de préparation de pentane-1,5-diamines 2,2-disubstituées, de la formule générale I 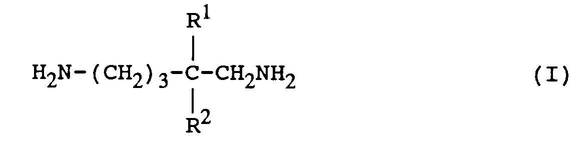 dans laquelle les substituants R¹ et R² représentent, indépendamment l'un de l'autre, chacun un radical alkyle en C₁ à C₁₀, alcényle en C₂ à C₁₀, ou forment en commun une chaîne alkylénique en C₄ à C₇, éventuellement une à cinq fois substituée par des radicaux alkyle en C₁ à C₄, au départ de 4-cyanobutanals 2,2-disubstitués de la formule générale II 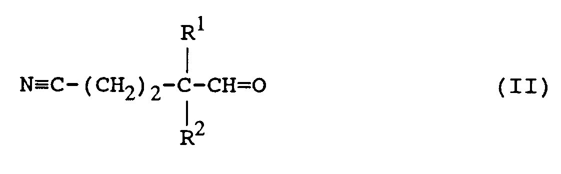 dans laquelle les substituants R¹ et R² possèdent les significations qui leur ont été attribuées ci-dessus, caractérisé en ce que, dans deux enceintes de réaction mutuellement séparées dans l'espace,
a) on fait réagir les 4-cyanobutanals de la formule II, dans une première enceinte de réaction, avec de l'ammoniac excédentaire, sur des catalyseurs hétérogènes acides, à des températures de 20 à 150°C et sous des pressions de 15 à 500 bars et
b) on hydrogène les produits de la réaction ainsi obtenus, dans une seconde enceinte de réaction, à l'aide d'hydrogène et en présence d'ammoniac excédentaire, sur des catalyseurs contenant du cobalt, du nickel, du ruthénium et/ou d'autres métaux nobles, avec des composants basiques ou sur des supports basiques, ou avec des composants basiques sur des supports neutres, ou des composants basiques, servant d'additif, au cours du processus d'hydrogénation, à des températures de 60 à 150°C et sous des pressions de 50 à 500 bars.

2. 2-Méthyl-2-n-propyl-pentane-1,5-diamine.

3. 2-n-Pentyl-2-n-propyl-pentane-1,5-diamine.
